Europäisches Patentamt

European Patent Office

Office européen des brevets

(11) Publication number: **0 108 294**
**B1**

(12) **EUROPEAN PATENT SPECIFICATION**

(45) Date of publication of patent specification: **06.02.91**

(21) Application number: **83110342.9**

(22) Date of filing: **17.10.83**

(51) Int. Cl.⁵: **C 01 B 21/14, C 07 C 51/41,
C 07 C 249/04, C 07 C 259/04**

(54) Preparation from hydroxylammonium sulfate of alcoholic hydroxylamine solutions and of oximes, hydroxamic acids and other hydroxylammonium salts via alcoholic hydroxylamine solutions.

(30) Priority: **01.11.82 US 437920**
**23.09.83 US 534292**
**23.09.83 US 534291**

(43) Date of publication of application:
**16.05.84 Bulletin 84/20**

(45) Publication of the grant of the patent:
**06.02.91 Bulletin 91/06**

(84) Designated Contracting States:
**BE CH DE FR GB IT LI NL SE**

(56) References cited:
**DE-B-1 247 284**
**US-A-2 483 252**
**US-A-3 420 621**

**JOURNAL OF ORGANIC CHEMISTRY, vol.
11, May 1946, pages 207-214, Washington, US;
C.D. HURD et al.: "Hydroxamic acids from
aliphatic dicarboxylic acids"**

(73) Proprietor: **ALLIED-SIGNAL INC. (a Delaware
corporation)**
**Columbia Road and Park Avenue P.O. Box 2245R
Morristown New Jersey 07960 (US)**

(72) Inventor: **Mathew, Chempolil Thomas**
**19 Openaki Road**
**Randolph New Jersey 07869 (US)**
Inventor: **Ulmer, Harry Edwards**
**26 Ellsworth Avenue**
**Morristown, New Jersey 07960 (US)**

(74) Representative: **Brock, Peter William et al
URQUHART-DYKES & LORD 91 Wimpole Street
London W1M 8AH (GB)**

Courier Press, Leamington Spa, England.

**Description**

Hydroxylamine, usually in the form of salts such as hydroxylammonium sulfate, hydroxylammonium chloride or the like is widely used as a reagent for preparing various industrial, speciality and pharmaceutical chemicals. Many products containing oxime or substituted hydroxylamine groups are not susceptible to production in aqueous media. Accordingly, such materials are normally prepared by reaction of solutions of hydroxylammonium chloride in organic solvents such as methanol with the organic precursor in the presence of sufficient base to neutralize the by-product HCl. Because hydroxylammonium sulfate (also called hydroxylamine sulfate) is not soluble in methanol, however, the cheaper sulfate reagent cannot be used to prepare these materials. Many other hydroxylammonium salts are more difficult to prepare than the sulfate (made by the Raschig process) and therefore would desirably be made from the sulfate. Proposals to do so in an ion exchange, exclusion or extraction column process are contained in US—A—4166842, 4147623 and 4202765.

Hydroxylammonium salts of organic acids are made in US—A—2483252 by reaction of hydroxylammonium sulfate or chloride with ammonium or alkali metal salts of such acid, added as such or made in situ, with the reaction temperature being usually 40—70°C. (see col. 2, lines 1—37).

A process for producing hydroxylammonium perchlorate is disclosed in US—A—3420621. A process for producing alcohol hydroxylamine slution is disclosed in DE—B—1247284.

A process has been discovered which enables solid hydroxylammonium sulfate to be used to provide hydroxylamine values in alcoholic solutions and to produce oximes, hydroxamic acids and other hydroxylammonium salts therefrom. Accordingly, the present invention in its first form includes a process comprising the steps:

(a) reacting an alcoholic solution of sodium hydroxide, or a sodium alkoxide having 1 to 5 carbons with solid hydroxylammonium sulfate, employing an alcohol having 1 to 3 carbons, at a temperature not greater than 30°C, employing a pressure and a time sufficient to produce a liquid phase having at least 50% of the hydroxylamine values of the hydroxylammonium sulfate, and

(b) separating the solid phase comprising sodium sulfate from the liquid phase.

In the simplest form, this process produces an alcoholic solution of free hydroxylamine with little or no water content (depending upon the base used) and a by-product solid phase containing sodium sulfate. Depending, however, upon how much base is used, the alcoholic solution produced may be more or less basic than free hydroxylamine. Furthermore, the alcoholic solution may be converted into various salts, including hydroxylammonium chloride and hydroxylammonium nitrate, preferably after separation of the solid phase and may also be reacted with an organic reagent to produce an oxime or substituted hydroxylamine or hydroxamic acid product before or after separating the solid phase.

In a second form of the invention, the above steps (a) and (b) are thus followed by:

(c) reacting the liquid phase with an acid other than sulfuric acid to produce an hydroxylammonium salt other than hydroxylammonium sulfate.

An alternative embodiment of the present invention comprises carrying out step (a) in the presence of an organic acid, and in step (b) the solid phase comprising sodium sulfate is separated from a liquid phase containing an organic hydroxylammonium salt corresponding to said organic acid, said liquid phase being substantially free of sulfate.

A further embodiment of the process comprises reacting the hydroxylamine values in the liquid phase with an organic reagent selected from aldehydes, ketones, acid chlorides and esters to form the corresponding aldoxime, ketoxime or hydroxamic acid product, and separating the solid phase comprising sodium sulfate from the liquid phase containing said corresponding product.

The organic reagent may be added (in this fourth form) before, during or after the reaction of step (a) and, if after step (a), then before or after the sodium sulfate is removed.

The reaction will not occur rapidly during step (a) even if the organic reagent is present because the conditions for rapid oximation (pH 5—8 and temperatures over 25°C) are not present during step (a).

The materials used in the process of the present invention are a base, an alcohol solvent and hydroxylammonium sulfate. Depending upon the form and product, other materials may be an aldehyde, a ketone, an ester, an acid chloride or an acid corresponding to the desired product salt. The hydroxylammonium sulfate is normally in solid form, preferably divided up into relatively fine powder or crystals, and may be produced in a variety of processes including, especially, that described in US—A—4349520. The alcohol may be any alkanol of 1—3 carbons, and especially methanol or ethanol, but also isopropanol and propanol when the base is an alkoxide. The base is sodium hydroxide, or a sodium alkoxide having 1 to 5 carbons such as sodium methoxide, ethoxide, isopropoxide, propoxide and butoxide. It is contemplated, however, that for any particular base, not all alcohols are suitable. Furthermore, for any particular base, specified conditions of temperature and/or pressure may be necessary to achieve the desired conversion of at least 50% of the hydroxylamine values from the solid hydroxylammonium sulfate to the liquid phase. The other acid may be any organic or inorganic acid other than sulfuric acid.

In the case of sodium hydroxide as base, any of the alcohols indicated above may be used as solvent. The preferred solvent for use with sodium hydroxide is methanol, with ethanol being slightly less preferred. It has been found that for both methanol and ethanol as solvent, the process of the present invention proceeds to higher conversions at lower temperatures. Thus the reaction temperature, while it

2

EP 0 108 294 B1

may be as high or about 30°C, is preferably no greater than 20°C and more preferably no greater than 10°C. Comparative Example 3, below, illustrates the significantly lower yields obtained at 35—40°C compared to those obtained at 22—25°C (e.g., Example 2) and at 5—10°C (e.g., Example 1). The concentration of sodium hydroxide in methanol or ethanol is not critical, but it is preferred to operate as near to the solubility limit of sodium hydroxide in the alcoholic solvent as possible without creating so viscous a solution that agitation becomes difficult. Larger amounts of the solvent may also be used if tolerable in subsequent reactions. Various of the examples illustrate the use of relatively concentrated methanolic and ethanolic solutions of sodium hydroxide in the present process. The amount of sodium hydroxide should be at least that required to neutralize 50% of the hydroxylammonium sulfate reacted, preferably at least that necessary to neutralize all of the hydroxylammonium sulfate. It is contemplated that greater amounts of sodium hydroxide than that stoichiometrically required may be used and, as indicated in Example 9 below, such excess sodium hydroxide may increase the reaction rate without detracting from reaction yields. Excess sodium hydroxide is normally to be used, however, only if the product alcoholic solution is to be used as a reagent in processes where more base would normally be charged at a later time. In other cases, the excess base can be neutralized before the solution is used further. Thus, the product solution can be formed at any desired pH such as from 5 to 12.

In using sodium hydroxide in ethanol as the solvent, lower temperatures are still preferred, with reaction being below 30°C, preferably below 20°C and more preferably no greater than 10°C. It appears, however, that the reaction in ethanol is less temperature dependent than the reaction in methanol (see Examples 11 and 12 below).

While isopropanol or propanol may be used as solvents with NaOH, the limited solubilities of the hydroxide and of the product hydroxylamine in these solvents makes these embodiments less preferred to those described above.

In using sodium hydroxide, pressure is not a critical factor since neither the base nor the solvent is very volatile.

Alkoxides, i.e. sodium methoxide, ethoxide, isopropoxide, propoxide, butoxide and pentylate may be used in place of the hydroxide, having the advantage of not producing water as by-product. Therefore, when a substantially water-free hydroxylamine solution is desired, these more expensive alkoxide bases should be used. Normally, the solvent will correspond to the anion (e.g., sodium methoxide in methanol), but mixed systems (e.g., sodium butoxide in methanol) may be used if the solvent later present (after the hydroxylamine-consuming reaction) is not to be recovered and recycled or can be distilled. While the anion may be larger than three carbons, the solvent is a 1—3 carbon alkanol (and is preferably methanol or ethanol) because free hydroxylamine is more soluble in these lower alkanols. If the product hydroxylammonium salt is organic (e.g. the acetate) and the anion is present initially (e.g. by reacting sodium acetate), then the solubility of free hydroxylamine becomes less critical and higher alcohol solvents, e.g. isopropanol or n-propanol, become more suitable.

Alkoxides are more expensive than hydroxides and are, therefore, normally not used unless the 3% or so water in the product solution of the above reactions of hydroxide cannot be tolerated for a particular use. The present invention, using alkoxides, still makes available the use of cheaper hydroxylammonium sulfate for such water-sensitive uses.

In each case, one preferred mode of conducting the reaction is to first dissolve (or slurry) the base in the alcohol and then react the alcoholic solution with hydroxylammonium sulfate. As illustrated by Examples 1 and 8, below, essentially identical results can be achieved either by adding the solid hydroxylammonium sulfate to the alcoholic solution or by adding the alcoholic base to the solid hydroxylammonium sulfate. Furthermore, it is contemplated that the two may be mixed in any conventional batch or continuous process scheme normally used to react a solid with a liquid. A less preferred method of conducting the present invention is to mix the base (solid) with the hydroxylammonium sulfate first, and then to add the alcohol. This scheme is less preferred because the process of dissolving the base in the alcohol (which is required before the reaction can occur) is normally an exothermic reaction. Since high temperatures are to be avoided it is desirable that the act of dissolving base in alcohol be conducted first, that the alcoholic solution be cooled and that the cooled alcoholic solution be reacted with the hydroxylammonium sulfate. Another less preferred method is to add the base slowly to hydroxylammonium sulfate slurried in alcohol.

In the second form of the invention, once the reaction between alcoholic base and hydroxylammonium sulfate is complete, or while it is proceeding, the alcoholic solution containing hydroxylamine values is further reacted with either a mineral acid (inorganic acid) or an organic acid. In one mode, this reaction is conducted after separating the by-product sulfate (e.g., sodium sulfate) from the alcoholic hydroxylamine solution. Thus, as illustrated in certain Examples below, the separated hydroxylamine-containing liquid phase is reacted with acids such as HCl, nitric acid, phosphoric acid, perchloric acid, oxalic acid, acetic acid, formic acid or benzoic acid to produce the corresponding hydroxylammonium salt (e.g., hydroxylammonium chloride, hydroxylammonium nitrate, dihydroxylammonium oxalate or trihydroxylammonium phosphate). In addition to these acids exemplified, one can use, for example, arsenic acid, fluoboric acid, propionic acid, toluenesulfonic acid, benzenesulfonic acid. In general, because of the ease of reacting alcohol hydroxylamine solutions, any such acid can be used even if the product is slightly unstable, such as is the case for the formate, nitrate and perchlorate. Excess base may be removed by neutralizing to pH 7—9

3

before separating the sulfate solids and introducing the other acids (as in Examples 25—28 and 31—36). If it is desired to recover such salt in solid form, the alcohol may be evaporated off (preferably under vacuum) or may be precipitated by the addition of a non-solvent for the salt such as a hydrocarbon. It is contemplated that such further reaction of hydroxylamine may be conducted prior to separating the by-product sulfate.

In the fourth form of the invention, as free hydroxylamine becomes available in the alcoholic solution, it may react with ketone or aldehyde (provided that the proper pH is reached). Reaction of alcoholic hydroxylamine with a mineral acid (inorganic acid) is preferably, however, conducted only after the by-product sulfate salt is removed. Organic acids, giving products which are in the liquid phase, are preferably reacted before removing the sulfate, but recovered from alcohol solvent after removing the solid sulfate phae. A convenient form of organic acid used in these instances is the ammonium or alkali metal salt of the organic acid (i.e., as indicated for the third form of the invention using basic organic salts). Such salts may be hydrated but are preferably not. With anhydrous salts, as with alkoxides, no water of neutralization is formed. In making organic hydroxylammonium salts, especially, it is also preferred that substantially no water be introduced into the reaction mixture with the alcohol: preferably the water:alcohol ratio is 0.05:1 or less, more preferably 0.01:1 or less and most preferably 0.001:1 or less. In general the only water added with the alcohol is that forming an azeotrope with recycled alcohol. In such event, especially if anhydrous alkoxides or organic salts are used, the water content of the product liquid phase will be particularly low and, therefore, the sulphate level in the hydroxylammonium salt produced will be very low.

The step of removing the by-product solid sulfate from the alcoholic solution containing hydroxylamine values may be carried out using any conventional technique for separating a solid from a liquid. Centrifugation, filtration, decantation and other conventional engineering steps are included. It is preferred that the recovered solid be washed with a solvent (such as the alcohol used for the solution) to remove adhered hydroxylamine-containing alcohol. Thereafter the solid may be dried, washed or recrystallized, treated in other ways to recover unreacted hydroxylammonium sulfate, or disposed of as initially separated.

The hydroxylammonium salt now in alcohol solution can be recovered or transferred to aqueous media in a variety of ways. Simple evaporative crystallization will produce solid salt of reasonably high purity, and recrystallization may be used to improve purity. The solid salt may then be redissolved in water in selected amounts to produce aqueous solutions of desired concentrations. For salts which are unstable in concentrated form (e.g. the perchlorate or nitrate), water can be added to the alcoholic solution, followed by evaporative concentration. In such event, the alcohol evaporated off (for recycle to step a) may be an azeotrope, which is approximately 1% water for methanol, approximately 4% water for ethanol and approximaely 15% water for isopropanol. These azeotropes can be broken by conventional techniques if a lower water level is desired for step a (so as to produce lower sulfate levels in the product).

In the fourth form of the invention, once the reaction between alcoholic base and hydroxylammonium sulfate is complete, or while it is proceeding, the alcoholic solution containing hydroxylamine values may be further reacted with an organic reagent such as an aldehyde or ketone. In one mode, this reaction is conducted after separating the by-product sulfate (e.g., sodium sulfate) from the alcoholic hydroxylamine solution. Thus, as illustrated in the Examples below, the alcoholic hydroxylamine solution is reacted with methyl ethyl ketone to produce methyl ethyl ketone oxime. Such reaction may be conducted at the pH normally used for the reaction involved, with pH between about 5 and about 8 used to convert ketones or aldehydes to oximes. It is contemplated that such further reaction of hydroxylamine may be conducted prior to separating the by-product sulfate, such as by having a ketone present in or added with the alcoholic sodium hydroxide solution or added after the free base is liberated but before the sulfate salt is filtered out. In such case, as free hydroxylamine becomes available in the alcoholic solution, it reacts with ketone or aldehyde (provided that the proper pH and temperature is reached).

The various modes of reaction can be illustrated for acetone, for example, which like most carbonyl compounds reacts rapidly with hydroxylamine values only at pH 5—8 and temperatures of at least about 25°C.

*Mode 1*

Step 1: Mix NaOH, MeOH, hydroxylammonium sulfate (HS) and acetone and stir at 5°C for approximately 5 hours.

Step 2: Adjust to pH about 8, raise temperature to 30°C and stir for 2 hours.

Step 3: Filter out Na$_2$SO$_4$.

Step 4: Evaporate solvent and recover acetone oxime crystals.

*Mode 2*

Same as Mode 1 with step 3 after adjusting to pH about 8 and before raising temperature to 30°C.

*Mode 3*

Step 1: Mix NaOH, MeOH and HS and stir at 5°C for about 5 hours.

Step 2: Add acetone, adjust pH to about 8, raise temperature to 30°C and stir for about 2 hours.

Step 3: Filter with Na$_2$SO$_4$.

Step 4: Evaporate solvent and recover acetone oxime crystals.

*Mode 4*

Same as Mode 3 with step 3 after adjusting to pH 8 and before raising temperature to 30°C.

Modes 1 and 3, wherein the $Na_2SO_4$ is filtered out last, are preferred for three reasons: first, the presence of $Na_2SO_4$ can help dry the liquid phase, even of by-product water from the oximation reaction of step (b); second, any grit or other solids entering during any reaction step will be present in the by-product sulfate salt rather than in the organic product crystals and, third, sulfate removal may be more complete with a larger and less polar organic liquid layer.

Organic reactants suitable for the present process include any that will react with the hydroxylamine values in the liquid phase after reaction step (a), with or without suitable pH and temperature adjustments. Suitable aldehydes which, on reaction, produce aldoximes, include formaldehyde, acetaldehyde, propionaldehyde, benzaldehyde, hydroxybenzaldehyde (such as salicylaldehyde) butyraldehyde and isobutyraldehyde. Suitable ketones which, on reaction, produce ketoximes include acetone, butanone (MEK), 3-pentanone (diethylketone), benzoin, acetophenone, benzophenone, o-hydroxybenzophenone and o-hydroxyacetophenone. Benzoquinonedioxime can be produced by the present process from p-nitroso-phenol. Hydroxamic acids can be produced by the present process from esters and acid chlorides such as ethyl acetate and benzoyl chloride.

The step of removing the by-product solid solvent from the alcoholic solution containing oxime or hydroxamic acid may be carried out using any conventional technique for separating a solid from a liquid. Centrifugation, filtration, decantation and other conventional engineering steps are included. It is preferred that the recovered solid be washed with a solvent (such as the alcohol used for the solution) to remove adhered hydroxylamine-containing alcohol. Thereafter the solid may be dried, washed or recrystallized, treated in other ways to recover unreacted hydroxylammonium sulfate, or disposed of as initially separated.

The product (oxime or hydroxamic acid) may be subjected to further reaction in the alcohol solvent or may be recovered therefrom by conventional techniques such as distillation, evaporative crystallization or precipitation with added non-solvent.

The present invention is illustrated by the following Examples, which are not intended to limit the invention:

## Example 1

A solution of methanolic sodium hydroxide was prepared by mixing sodium hydroxide pellets (17.2 g; 0.43 mol) with absolute methanol (150 mL) in a 250 mL Erlenmeyer flask.

In the meantime a 500 mL 3-necked flask was fitted with a thermometer, dropping funnel and nitrogen inlet (inert atmosphere) and a magnetic stirring bar (PTFE-coated, $1\frac{1}{2}$ inches or 3.8 cm long) was placed in it. Solid hydroxylamine sulfate (35 g; 0.213 mol) was placed in the flask with methanol (50 mL) and the flask was placed in an ice-water bath over a stir plate. With vigorous stirring, the methanolic NaOH solution was added slowly (over 5 minutes) using the dropping funnel, maintaining a reaction mixture temperature below 10°C. After the addition was complete, stirring was continued to $1\frac{1}{2}$ hours more with cooling (5—10°C). A white slurry resulted and this was filtered over a Buchner funnel and the cake was washed with more methanol (25 mL). The clear and colorless filtrate (pH 12.5) was analyzed for free hydroxylamine by mixing with known excess of methyl ethyl ketone (MEK) (40 g) and adjusting the pH to 7 with concentrated $H_2SO_4$ (2.5 g). Methyl ethyl ketoxime formed was determined by gas chromatography to correspond to free hydroxylamine (87.4% yield).

The white filter cake (34.2 g) of sodium sulfate was analyzed for remaining hydroxylamine sulfate by dissolved in water (150 mL) and mixing with excess of MEK (40 g) and titrating with 50% NaOH solution (3.9 g) to pH 7. The amount of hydroxylamine sulfate left in the cake represented 11.4% of the total.

## Examples 2—24

With the variations in reagents and conditions shown in Table 1, Example 1 was repeated, with the results (expressed as percentage of hydroxylamine values fed present in the solution and in the filter cake) as indicated in Table 1. The numbers of Comparative Examples are prefixed by the reference "C".

## Table 1

| Example | Base | Solvent | Temperature | Time (hours) | Yield (%) | % In Cake |
|---------|------|---------|-------------|--------------|-----------|-----------|
| 1 | NaOH | MeOH | 5–10°C | 1.5 | 87.4% | 11.4% |
| 2 | NaOH | MeOH | 22–25°C | 2 | 71.2% | — |
| C3 | NaOH | MeOH | 35–40°C | 1.5 | 18.8% | 76.5% |
| 4 | NaOH | MeOH | 5–10°C | 6 | 90.7% | 0.9% |
| 5 | NaOH | MeOH | 10°C | 6 | | |
| | | then | 22°C | 12 | 80.5% | trace |
| 6 | NaOH | MeOH | <10°C | 4 | 91.6% | 7.6% |
| 7* | NaOH | MeOH | 5°C | 4 | 82.1% | 17.3% |
| 8** | NaOH | MeOH | 5–10°C | 1.5 | 86.6% | 10.4% |
| 9* | NaOH | MeOH | 7–10°C | 1 | 78.3% | 12.2% |
| 10** | NaOH | MeOH | 5–10°C | 2 | 70.8% | 27.9% |
| 11 | NaOH | EtOH | 5–10°C | 3 | 88.3% | 4.7% |
| 12 | NaOH | EtOH | 22–25°C | 3 | 89.1% | 2.9% |
| C13 | LiOH*** | MeOH | 10°C | 1.5 | 45.8% | 42.6% |
| C14 | LiOH*** | MeOH | 22–24°C | 3 | 53.8% | 13.1% |
| C15 | KOH | MeOH | 10°C | 3 | <1% | almost all |
| C16 | KOH | MeOH | 22–25°C | 1 | <5% | almost all |
| C17 | KOH | EtOH | 22–25°C | 3 | 77.9% | 16.6% |
| C18 | KOH | EtOH | 5–10°C | 3 | 70.0% | 24.1% |
| C19 | $NH_3$ | MeOH | about 20°C | 3 | 46.7% | 42.4% |
| C20** | $NH_3$ | MeOH | 5–10°C | 3 | 17.1% | 78.5% |
| C21** | $NH_3$ | MeOH | 10°C | 3 | 10.0% | 82.8% |
| C22** | $NH_3$ | MeOH | 40–50°C | 2 | 5.7% | 84.0% |
| C23** | $NH_3$ | EtOH | 19°C | 3 | 13.9% | 76.7% |
| C24** | NaOH | $Et(OH)_2$ | 20–25°C | 2 | | |
| | | then | 30°C | 1 | 7.4% | 75.3% |

\* The indicated examples used larger molar amounts of at least one reagent and/or larger volumes of alcohol solvent as follows:

| Example | Base (mol) | | Total Alcohol | | HS(mol) |
|---------|------------|------|---------------|--------|---------|
| 7 | NaOH | 2.58 | MeOH | 978mL | 1.28 |
| 9 | NaOH | 0.86 | MeOH | 288mL | 0.213 |

Comparative Example 19 — ammonia gas bubbled into a mixture of 0.213 mol HS, 200 mL MeOH and 1.0 mL water. Repeated until pH stayed above 9.0;

Comparative Examples 20—22 — 11.6% $NH_3$ solution (10.2 g $NH_3$) in MeOH added to 0.213 mol HS in 100 mL MeOH. Autoclave used for Comparative Examples 21 and 22.

Comparative Example 23 — 5.7% $NH_3$ solution in ethanol (8.55 g $NH_3$) added to 0.213 mol HS in 50 mL ethanol.

*** $LiOH \cdot H_2O$

## Preparation of Hydroxylammonium Salts

The preparation of salts from alcoholic hydroxylamine solutions are illustrated in the following examples:

| Example | salts | |
|---------|-------|---|
| 25 | Hydroxylammonium | Chloride |
| 26 | " | Nitrate |
| 27 | " | Phosphate |
| 28 | " | Oxalate |
| 31 | " | Acetate |
| 32 | " | Acetate |
| 33 | " | Benzoate |
| 34 | " | Formate |
| 35 | " | Formate |
| 36 | " | Perchlorate |

### Example 25

In a 500 mL 3-necked flask was placed hydroxylamine sulfate (70 g; 0.43 mol) with methanol (50 mL). To this was added with stirring using magnetic stirring bar a solution of NaOH pellets (34.4 g; 0.86 mol) in methanol (300 mL) over 15 minutes with cooling in an ice-water bath. Stirring was continued over 3 hours at temperatures ranging from 2 to 7°C. The pH of the slurry was recorded (11.2) and conc. $H_2SO_4$ (1.5 g) was added dropwise until pH 8.0 was reached.

The white slurry was filtered and the cake washed on the filter with more ethanol. The total filtrate (323 g) was analyzed potentiometrically and found to contain free hydroxylamine corresponding to 85.2% of the starting sulfate.

The filter cake (69 g) was analyzed for hydroxylamine left behind (10.9%).

The methanolic solution of hydroxylamine was placed in a 500 mL Erlenmeyer containing a magnetic stirring bar and the flask in turn was placed in an ice bath over a stir plate. HCl gas was slowly bubbled into the solution with stirring and maintaining the temperature at 20—25°C. HCl solution was continued until the pH dropped from 8.0 to 2.8. The solution was then placed in a 1 liter round bottom flask and evaporated to dryness under reduced pressure. White crystalline solid of hydroxylamine hydrochloride (49.9 g) was collected (M.P. 154.5°C) yield 84.1%.

### Example 26

A solution of sodium methoxide in methanol produced by dissolving sodium (10 g; 0.435 mol) in methanol (150 mL) was stirred with hydroxylamine sulfate (35 g; 0.213 mol) in ice-water bath (5—10°C) over 2 hours. The slurry filtered and the clear methanolic filtrate with cake-wash (pH 9.2) was mixed with conc. $H_2SO_4$ (0.8 g) to pH 8.0. The thin white solid produced was filtered off and the clear filtrate was placed in 500 mL Erlenmeyer with a magnetic stirring bar. While cooling in ice-water bath and stirring, conc. $HNO_3$ (35.5 g) was added till the pH reached 2.8. The clear methanolic solution of hydroxylamine nitrate (273.3 mL) was found to contain 37.99 g $NH_2OH \cdot NHO_3$ (13.9 g in 100 mL). Overall yield 92.7%.

### Example 27

A solution of hydroxylamine in methanol prepared as in Example 7 was used. The solution (100 mL containing 4.84 g $NH_2OH$) was placed in a 250 mL 3-neck flask fitted with thermometer and a dropping funnel and containing a magnetic stirring bar. With stirring and cooling in an ice-water bath (5°C) 85% orthophosphoric acid (8.0 g; 0.069 mol) was slowly added until the pH of the solution dropped from 11.8 to 8.0, and a bulky white slurry was produced. After stirring at 5°C for 15 minutes more, the solid was collected by filtration (11.8 g crude cake). It was recrystallized from hot water, and white crystalline hydroxylammonium phosphate (8.2 g on drying) was collected. Yield 85.4% (M.P. 175°C with decomposition).

### Example 28

A solution of hydroxylamine in methanol (680 mL) as in Example 27 was used (pH 11.8). Conc. $H_2SO_4$ was slowly added to adjust the pH to 8.0 and the thin white precipitate formed was filtered off and the clear filtrate placed in a 1 liter Erlenmeyer flask. A magnetic stirring bar was introduced and the flask was placed in an ice-water bath. Oxalic acid (45 g; 0.5 mol) dissolved in methanol (100 mL) was slowly added with cooling and stirring. A thick white slurry was produced and this was filtered and the crude white solid (84.8 g) was collected. A portion (25 g) of this solid was recrystallized from hot water to produce white crystalline hydroxylammonium oxalate (M.P. 192°C with decomposition). Yield 94.6%.

### Example 29

A 500 mL 3-necked flask was fitted with a thermometer, reflux-condenser and drying tube. Freshly cut sodium (10.0 g; 0.435 mol) was placed in the flask and absolute methanol (175 mL) was carefully added with cooling. After the sodium was completely dissolved in methanol forming a clear solution of sodium methoxide, solid hydroxylamine sulfate (35 g; 0.213 mol) was added with cooling over 2 minutes. No significant exotherm was noticed. The mixture was stirred with cooling (10°C) in ice-water bath using a magnetic stirring bar over a stir plate for one hour. Subsequently, cooling was removed and vigorous stirring continued at ambient temperature for 2 hours or more. By this point a white slurry had formed, and this was filtered and the cake washed using more methanol. The total filtrate (162 g) was analyzed potentiometrically and found to contain hydroxylamine corresponding to 87.5% yield. The filtrate was virtually free of water (<0.5% $H_2O$).

The white solid (32 g) was dissolved in water and analyzed for unused hydroxylamine sulfate (1.6%).

### Comparative Example 30

In a 500 mL 3-necked flask, fitted with thermometer, reflux condenser, and drying tube was placed absolute methanol (100 mL) and freshly-cut potassium (8.4 g; 0.215 mol) was added piece-by-piece with cooling in ice-bath and a clear solution of potassium methoxide in methanol was produced. To this solution was added with vigorous stirring crystalline hydroxylamine sulfate (17.5 g; 0.107 mol) and stirring was continued at ambient temperature for 3 hours. No noticeable change (no milkiness) was found to be developing.

The slurry was filtered and the filter cake was washed with more methanol. The total filtrate (125 g) was analyzed potentiometrically and found to contain virtually no hydroxylamine (<0.3% yield). The crude filter cake (18 g) which appeared crystalline (similar to the starting hydroxylamine sulfate) was dissolved in water (75 mL) and analyzed and found to contain over 95% of the starting hydroxylamine sulfate.

### Example 31
#### Preparation of Hydroxylammonium Acetate

A 2 liter 3-neck flask with baffles was fitted with thermometer, overhead agitator and a dropping funnel. A mixture of solid hydroxylamine sulfate (210 g, 1.28 mol) and absolute methanol (400 mL) was placed in the flask and vigorously agitated with cooling in ice bath (0—5°C). A solution of sodium hydroxide pellets (103 g; 2.58 mol) in absolute methanol (700 mL) was slowly added using the dropping funnel. The slurry was stirred vigorously over 7 hours with continued cooling; and concentrated $H_2SO_4$ (5.8 g) was added to adjust the pH to 8.8. The white slurry was filtered and the clear filtrate (1165 mL) analyzed (potentiometric titration) and found to contain 5.99 g of hydroxylamine per 100 mL of the solution.

In a 1 liter Erlenmeyer flask provided with a PTFE-coated magnetic stirring bar was placed a portion (690 mL) of the methanolic hydroxylamine solution. With cooling in ice-water bath (5—10°C) and stirring glacial acetic acid (75 g; 1.25 mol) was slowly added over 20 minutes. The clear solution (pH 5.6) was then stirred at ambient temperature for 1 hour and was evaporatd on the rotovap to remove methanol under reduced pressure. A colorless and very viscous liquid was obtained (116 g), which on standing crystallized into a white crystalline solid. Yield (99.5%).

### Example 32
#### Preparation of Hydroxylammonium Acetate

A hydroxylamine solution in methanol prepared as in example 31 containing 4.1 g $NH_2OH$ per 100 mL of solution was used. Glacial acetic acid (30 g; 0.43 mol) was diluted with distilled water (30 g) and this 50% aqueous acetic acid was added slowly with stirring (magnetic stirring bar) and cooling (ice bath) to the hydroxylamine solution (350 mL; 0.43 mols $NH_2OH$) in a 500 mL Erlenmeyer flask. The pH was 5.6. The clear solution was carefully evaporated to remove methanol and an aqueous solution of hydroxylamine acetate (75 g) of approximately 60% concentration was obtained in excellent yield as a colorless liquid.

### Example 33
#### Preparation of Hydroxylammonium Benzoate

A solution of hydroxylamine in methanol prepared as in example 31 was used (4.1 g $NH_2OH$ in 100 mL solution). The hydroxylamine solution (41 mL) was placed in a 250 mL Erlenmeyer flask furnished with magnetic stirring bar and was kept cooled in ice-water bath (about 10°C). The pH was 7.6. The solution of benzoic acid (6.1 g; 0.05 mol) in absolute methanol (50 mL) was slowly added with stirring (pH 6.3). The clear mixture was then stirred for one hour at room temperature and then carefully evaporated to dryness. Hydroxylamine benzoate was collected as a white crystalline solid (7.5 g). Yield 96.8%.

## Example 34

Preparation of Hydroxylammonium Formate

A methanolic solution of hydroxylamine (820 mL; at 5.56 g/100 mL) prepared as in example 31 was placed in a 2 liter Erlenmeyer flask furnished with a magnetic stirring bar. This was kept cooled in ice-water bath (about 10°C) with stirring and a solution of formic acid (63.8 g; 1.38 mol) in distilled water (77 g) was slowly added. After completion of addition it was stirred further for an hour without cooling and then transferred to a 2 liter round bottom flask and the methanol carefully evaporated off on a rotary evaporator. A clear and colorless solution of aqueous hydroxylamine formate was collected (177.5 g; 61% solution).

## Example 35

Preparation of Hydroxylammonium Formate

A solution of hydroxylamine in methanol, prepared as in example 31, containing 4% of $NH_2OH$ (potentiometric titration) was used. A 500 mL Erlenmeyer flask containing a PTFE-coated magnetic stirring bar was placed in an ice bath and the hydroxylamine solution (225 mL) was added to it with stirring (temp. 5°C). Formic acid (14.5 g; 0.31 mol) was then added slowly to it. Temperature rose to 20°C by the end of the addition (pH 5). The clear solution was stirred for one half-hour more without cooling and then evaporated under reduced pressure (rotovap) in a 500 mL round bottom flask. A white crystalline solid was collected (19.8 g; yield 92.8%; M.P. 74—75°C).

## Example 36

Preparation of Hydroxylammonium Perchlorate

A solution of 50% perchloric acid in water was prepared by mixing 70% perchloric acid (Fisher Scientific) with distilled water.

A methanolic hydroxylamine solution (300 mL; at 5.27 g in 100 mL solution) was placed in a 500 mL Erlenmeyer flask and it was cooled in ice bath with stirring using a magnetic PTFE-coated stirring bar. The perchloric acid solution was slowly added (99 g of the 50% solution) to pH 3.15. After stirring further for one half hour at room temperature, the clear solution was transferred to a 500 mL round bottom flask and the methanol carefully distilled off under reduced pressure (rotovap) without any heating. A clear liquid (112.5 g) was collected which was analyzed by potentiometric titration to be 56% solution of hydroxylammonium perchlorate (yield 98.5%).

## Example 37

Preparation of Benzophenone Oxime

Sodium hydroxide pellets (103 g; 2.58 mol) were dissolved with stirring in absolute methanol (750 mL) at ambient temperature. The solution was placed in a dropping funnel placed on a 3-neck, 2 liter, baffled round bottom flask fitted with thermometer and an overhead agitator. In the flask was placed solid hydroxylamine sulfate (210 g; 1.28 mol) mixed with absolute methanol (450 mL). With vigorous agitation and cooling in an ice bath (0—5°C), the methanolic sodium hydroxide solution was added over about 30 minutes. The white slurry was stirred further with cooling for 5 hours more and pH adjusted from pH 12 to 9 using concentrated $H_2SO_4$ (12 g) and filtered. The filter cake of sodium sulfate was washed on the funnel with absolute methanol (50 mL). The total filtrate (1280 mL, clear, colorless solution) was analyzed for free hydroxylamine base and found to contain 5.27 g/100 mL solution.

A 500 mL 3-neck flask was fitted with thermometer, dropping funnel and nitrogen blanket. Benzophenone (36.5 g; 0.2 mol) in absolute methanol (100 mL) was placed in the flask and cooled in an ice bath (5°C) with stirring using a magnetic stirring bar. Hydroxylamine solution (133 mL) was slowly added and after completion of addition (15 minutes), the mixture was heated at reflux (65°C). PH 8.6. After a total of 18 hours of heating under reflux, it was cooled and transferred to a 500 mL round bottom flask and concentrated on a rotovap. On cooling in ice bath, it was filtered and benzophenone oxime was collected as white crystalline solid (32.5 g; 89% yield). Purity, >97%.

The product was kept sealed in a clear jar purged with nitrogen and it remained stable indefinitely.

## Example 38

Preparation of Benzophenone Oxime

Benzophenone (182 g; 1.0 mol) mixed with absolute methanol (400 mL) was placed in a 2-liter 3-neck flask (baffled) fitted with over-head stirrer. To the solution was added hydroxylamine sulfate (120 g; 0.73 m) and maintained vigorously stirred with cooling in ice bath (5°C). A solution of sodium hydroxide pellets (60 g; 1.50 mol) in absolute methanol (400 mL) was then added slowly with continued agitation. After stirring for 6 hours with cooling, it was heated under reflux (67°C). PH of the mixture was 11.2. Heating at reflux continued for a total of 12 hours and the solution filtered while still hot.

The clear filtrate was then further concentrated on the rotovap and cooled and filtered to collect benzophenone oxime (174 g; 88.3%). Purity: about 98%. Major impurity was benzophenone (gas chromatography).

The oxime sample remained stable for over a long period of time in a bottle purged with nitrogen and tight-sealed with plastic tape. On the other hand, a portion of the same sample kept in a similar bottle loosely closed turned brown and eventually to a liquid after about 10 days.

## Example 39

### Preparation of Acetone Oxime

A 2-liter 3-neck flask with baffles was fitted with over-head stirring and thermometer and dropping funnel. Hydroxylamine sulfate (210 g; 1.28 mol) was placed in a flask mixed with absolute methanol (400 mL) and stirred vigorously with cooling in ice bath (5°C). A solution of sodium hydroxide pellets (103 g; 2.58 mol) in absolute methanol (800 mL) was added from the dropping funnel slowly over 30 minutes. Stirring with cooling was continued over 5 hours. PH of the white slurry was adjusted to 6.5 using Conc. $H_2SO_4$ (108 g). Acetone (148.5 g; 2.56 mol) was added to the slurry slowly and stirring continued for 1 hour more.

The white slurry was filtered to remove sodium sulfate and the clear filtrate concentrated on the rotovap. White crystalline solid of acetone oxime was collected (168 g) as the first crop. The colorless solution furnished a second crop of acetone oxime crystals (13.5 g) on further evaporation and cooling. Total yield 97.4%. Purity >99% (gas chromatography).

## Example 40

### Preparation of Acetophenone Oxime

In a 500 mL 3-neck flask was placed solid hydroxylamine sulfate (28 g; 0.17 mol) and absolute methanol (100 mL) was added to it. The slurry was stirred vigorously using a magnetic bar over a stir plate and a solution of sodium hydroxide pellets (14 g; 0.35 mol) in absolute methanol (175 mL) was then added slowly with cooling in ice bath (<5°C). At the completion of addition of the solution, acetophenone (40 g; 0.33 mol) was added and stirring in the cold was continued for 5 hours. Concentrated $H_2SO_4$ (1.8 g) was added to adjust the pH of the solution to 6.5. Methanol (50 mL) was added to the slurry and the mixture heated (40°C) for 2 hours and then filtered hot. The clear filtrate (colorless) was concentrated under reduced pressure and crystalline acetophenone oxime was collected after cooling and filtration. Yield, 40.5 g (90.8%). Purity >98% of isomeric mixture.

## Example 41

### Preparation of Acetone Oxime

In a 100 mL Erlenmeyer flask furnished with a magnetic stirring bar was placed a methanolic hydroxylamine solution (50 mL; 4.68 g $NH_2OH$ per 100 mL) prepared as in Example 25. Cooling in ice bath, the temperature was maintained at about 10°C and acetone (4 g; 0.069 mol) was added slowly with stirring. After stirring for 15 minutes more without cooling the clear, colorless solution (pH 7.3) was placed in a 200 mL round bottom flask and carefully evaporated (rotovap) and a crystalline solid collected (6.2 g). Gas chromatography showed that it was virtually pure acetone oxime. Yield 97.7%.

## Claims

1. A process comprising the steps of:

(a) reacting an alcoholic solution of sodium hydroxide, or of a sodium alkoxide having 1 to 5 carbons with solid hydroxylammonium sulfate, employing an alcohol having 1 to 3 carbons, at a temperature not greater than 30°C, employing a pressure and a time sufficient to produce a liquid phase having at least 50% of the hydroxylamine values of the hydroxylammonium sulfate; and

(b) separating the solid phase comprising sodium sulfate from the liquid phase.

2. A process according to Claim 1 characterised in that the sodium alkoxide is sodium methoxide.

3. A process according to Claim 1 or 2 characterised in that the alcohol is methanol.

4. A process according to Claim 1 or 2 characterised in that the alcohol is ethanol.

5. A process according to any one of Claims 1 to 4 characterised in that step (a) is conducted at a temperature not greater than 20°C.

6. A process according to any one of Claims 1 to 4 characterised in that step (a) is conducted at a temperature not greater than 10°C.

7. A process according to any one of Claims 1 to 6 characterised by (c) reacting the separated liquid phase with an acid other than sulfuric acid to produce an hydroxylammonium salt other than hydroxylammonium sulfate.

8. A process according to Claim 7 characterised in that the other acid is an organic acid.

9. A process according to Claim 7 characterised in that the other acid is a mineral acid.

10. A process according to any one of Claims 1 to 6 characterised in that step (a) is carried out in the presence of an organic acid, and in step (b) the solid phase comprising sodium sulfate is separated from a liquid phase containing an organic hydroxylammonium salt corresponding to said organic acid, said liquid phase being substantially free of sulfate.

11. A process according to any one of Claims 1 to 6, characterised in that the hydroxylamine values in the liquid phase are reacted with an organic reagent selected from aldehydes, ketones, acid chlorides and esters to form the corresponding aldoxime, ketoxime or hydroxaminic acid product, and separating the solid phase comprising sodium sulfate from the liquid phase containing said corresponding product.

# EP 0 108 294 B1

## Patentansprüche

1. Verfahren mit den Verfahrensstufen
(a) Umsetzen einer alkoholischen Lösung von Natriumhydroxid oder eines Natriumalkoxids mit 1 bis 5 Kohlenstoffatomen mit festem Hydroxylammoniumsulfat unter Verwendung eines Alkohols mit 1 bis 3 Kohlenstoffatomen bei einer Temperatur von nicht mehr also 30°C, unter Verwendung eines Drucks und eines Zeitraums, der ausreicht, um eine Flüssigphase mit wenigstens 50% der Hydroxylaminwerte des Hydroxylammoniumsulfats zu erhalten, und
(b) Abtrennen der Natriumsulfat enthaltenden Festphase von der Flüssigphase.

2. Verfahren nach Anspruch 1, dadurch gekennzeichnet, daß das Natriumalkoxid Natriummethoxid ist.

3. Verfahren nach Anspruch 1 oder 2, dadurch gekennzeichnet, daß der Alkohol Methanol ist.

4. Verfahren nach Anspruch 1 oder 2, dadurch gekennzeichnet, daß der Alkohol Ethanol ist.

5. Verfahren nach einem der Ansprüche 1 bis 4, dadurch gekennzeichnet, daß die Verfahrensstufe (a) bei einer Temperatur von nicht mehr als 20°C ausgeführt wird.

6. Verfahren nach einem der Ansprüche 1 bis 4, dadurch gekennzeichnet, daß die Verfahrensstufe (a) bei einer Temperatur von nicht mehr als 10°C ausgeführt wird.

7. Verfahren nach einem der Ansprüche 1 bis 6, dadurch gekennzeichnet durch (c) Umsetzen der abgetrennten Flüssigphase mit einer anderen Säure als Schwefelsäure, um ein anderes Hydroxylammoniumsalz als Hydroxylammoniumsulfat zu erzeugen.

8. Verfahren nach Anspruch 7, dadurch gekennzeichnet, daß die andere Säure eine organische Säure ist.

9. Verfahren nach Anspruch 7, dadurch gekennzeichnet, daß die andere Säure eine Mineralsäure ist.

10. Verfahren nach einem der Ansprüche 1 bis 6, dadurch gekennzeichnet, daß die Verfahrensstufe (a) in Gegenwart einer organischen Säure ausgeführt wird und in der Verfahrensstufe (b) die Natriumsulfat enthaltende Festphase von einer ein der organischen Säure entsprechendes organisches Hydroxylammoniumsalz enthaltenden Flüssigphase, wobei die Flüssigphase im wesentlichen frei von Sulfat ist, abgetrennt wird.

11. Verfahren nach einem der Ansprüche 1 bis 6, dadurch gekennzeichnet, daß die Hydroxylaminwerte in der Flüssigphase mit einem aus Aldehyden, Ketonen, Säurechloriden und Estern ausgewählten organischen Reagenz umgesetzt werden, um das entsprechende Aldoximketoxim oder Hydroxaminsäureprodukt zu bilden, und Abtrennen der Natriumsulfat enthaltenden Festphase von der das entsprechende Produkt enthaltenden Flüssigphase.

## Revendications

1. Procédé comprenant les étapes consistant à:
(a) faire réagir une solution alcoolique d'hydroxyde de sodium, ou d'un alcoolate de sodium ayant 1 à 5 atomes de carbone avec du sulfate d'hydroxylammonium solide, à employer un alcool ayant 1 à 3 atomes de carbone à une température non supérieure à 30°C, à employer une pression et un temps suffisants pour produire une phase liquide ayant au moins 50% des valeurs en hydroxylamine du sulfate d'hydroxylammonium, et
(b) séparer le phase solide comprenant le sulfate de sodium de la phase liquide.

2. Procédé selon la revendication 1, caractérisé en ce que l'alcoolate de sodium est le méthylate de sodium.

3. Procédé selon la revendication 1 ou 2, caractérisé en ce que l'alcool est le méthanol.

4. Procédé selon la revendication 1 ou 2, caractérisé en ce que l'alcool est l'éthanol.

5. Procédé selon l'une quelconque des revendications 1 à 4, caractérisé en ce que l'étape (a) est effectuée à une température non supérieure à 20°C.

6. Procédé selon l'une quelconque des revendications 1 à 4, caractérisé en ce que l'étape (a) est effectuée à une température non supérieure à 10°C.

7. Procédé selon l'une quelconque des revendications 1 à 6, caractérise par l'étape (c) consistant à faire réagir la phase liquide séparée avec un acide autre que l'acide sulfurique pour produire un sel d'hydroxylammonium autre que le sulfate d'hydroxylammonium.

8. Procédé selon la revendication 7, caractérisé en ce que l'autre acide est un acide organique.

9. Procédé selon la revendication 7, caractérisé en ce que l'autre acide est un acide minéral.

10. Procédé selon l'une quelconque des revendication 1 à 6, caractérisé en ce que l'étape (a) est effectuée en présence d'un acide organique, et en ce que dans l'étape (b) la phase solide comprenant du sulfate de sodium est séparée d'une phase liquide contenant un sel d'hydroxylammonium organique correspondant audit acide organique, la phase liquide étant sensiblement exempte de sulfate.

11. Procédé selon l'une quelconque des revendications 1 à 6, caractérisé en ce que les valeurs en hydroxylamine dans la phase liquide sont amenées à réagir avec un réactif organique choisi parmi les aldéhydes, les cétones, l'acide chlorhydrique et les esters de manière à former le produit correspondant d'aldoxime-cétoxime ou d'acide hydroxamique, et l'étape de séparation de la phase solide comprend du sulfate de sodium provenant de la phase liquide comportant ce produit correspondant.